# FASCICULE DE BREVET EUROPEEN

(11) **EP 0 620 723 B1**
(45) Date de publication et mention de la délivrance du brevet: **15.04.1998**
(21) Numéro de dépôt: 94900188.7
(22) Date de dépôt: 12.11.1993
(51) Int. Cl.: A61K 7/043, A61K 7/06, C08F 255/02

(54) **COMPOSITION COSMETIQUE FILMOGENE A BASE D'UN COPOLYMERE CHLORE GREFFE RESULTANT DU GREFFAGE D'UNE POLYOLEFINE CHLOREE ET DE MONOMERES INSATURES DU TYPE ACRYLIQUE, STYRENIQUE ET/OU VINYLIQUE**
FILMBILDENDE KOSMETISCHE ZUSAMMENSETZUNG AUF BASIS VON EINEM CHLORIERTEN PFROPTCOPOLYMER, DAS AUS DER PFROPFUNG VON CHLORIERTEM POLYOLEFIN, UND UNGESAETTIGEN AKRYLAT, STYREN UND/ODER VINYLMONOMEREN RESULTIERT
FILM-FORMING COSMETIC COMPOSITION BASED ON A GRAFTED CHLORINATED COPOLYMER RESULTING FROM THE GRAFTING OF A CHLORINATED POLYOLEFIN AND ACRYLIC, STYRENE AND/OR VINYL TYPE UNSATURATED MONOMERS

(30) Priorité: 12.11.1992 FR 9213600
(43) Date de publication de la demande: 26.10.1994
(73) Titulaire: L'OREAL, 75008 Paris (FR)
(72) Inventeur: SER, Jean-Claude, F-94150 Chevilly Larue (FR)
(74) Mandataire: Stalla-Bourdillon, Bernard
(86) Numéro de dépôt international: FR9301110
(87) Numéro de publication internationale: WO9410966

(56) Documents cités:
- EP-A- 0 038 004
- GB-A- 2 073 229
- GB-A- 2 207 143
- GB-A- 2 254 082
- US-A- 4 283 384
- US-A- 4 683 007

## Description

La présente invention a pour objet de nouvelles compositions cosmétiques filmogènes contenant dans un véhicule approprié un copolymère chloré greffé résultant du greffage d'une polyoléfine chlorée et de monomères insaturés de type acrylique, styrénique et/ou vinylique.

Les polymères et copolymères synthétiques constituent des ingrédients essentiels d'un grand nombre de compositions cosmétiques filmogènes telles que des compositions de fixation de la chevelure, par exemple des laques et lotions de mises en plis ou des vernis à ongles.

De très nombreux polymères et copolymères ont déjà été proposés mais peu d'entre eux sont susceptibles d'être utilisés indifféremment dans des compositions cosmétiques aussi différentes que des produits capillaires pour la fixation dès cheveux et des vernis à ongles.

On a maintenant découvert qu'une certaine classe de copolymères résultant du greffage de polyoléfines chlorées par des monomères insaturés conduisait à des compositions cosmétiques de nature très variée, et que ceux-ci apportaient une amélioration des propriétés cosmétiques, en particulier une meilleure adhérence et brillance sur les matières kératiniques telles que les cils et les cheveux, ou encore sur les ongles.

Il a été tout particulièrement constaté que par l'emploi de tels copolymères dans des vernis à ongles, l'adhérence du vernis était améliorée de façon particulièrement significative.

La présente invention a donc pour objet une composition cosmétique filmogène contenant dans un véhicule cosmétique approprié au moins un copolymère chloré greffé résultant du greffage de 5 à 20 % d'une polyoléfine chlorée avec 80 à 95% de monomères insaturés du type acrylique, styrénique et/ou vinylique, monomères pouvant être choisis parmi les monomères oléophiles en une proportion maximum de 95 % et les monomères hydrophiles en une proportion maximum de 40%.

Selon l'invention, les polyoléfines chlorées conduisant à l'obtention du copolymère chloré greffé se caractérisent par un poids moléculaire moyen compris entre 8.000 et 140.000 et une teneur en chlore comprise entre 15 et 40 %.

Parmi les monomères insaturés, ceux du type oléophile sont plus particulièrement le styrène et les acrylates et méthacrylates d'alkyle en particulier de butyle. Parmi les monomères insaturés du type hydrophile, on peut citer l'acide acrylique, l'acide méthacrylique, les acrylates ou méthacrylates de 2-hydroxyéthyle ou de 2-hydroxypropyle.

Lorsque le copolymère chloré greffé est plus particulièrement destiné à la réalisation d'une composition cosmétique sous forme d'un vernis à ongles, les monomères insaturés sont du type oléophile alors que lorsque le copolymère chloré greffé est destiné à une composition cosmétique capillaire, les monomères insaturés sont du type hydrophile et plus particulièrement de l'acide acrylique ou méthacrylique.

Selon une forme de réalisation préférée, les monomères insaturés sont sous forme d'un mélange constitué de styrène, de méthacrylate de butyle, d'acide méthacrylique et de méthacrylate de 2-hydroxyéthyle.

Les copolymères chlorés greffés tels que définis ci-dessus, présents dans les compositions cosmétiques selon l'invention, sont obtenus selon les procédés classiques de greffage des polymères, qui consistent à soumettre une polyoléfine chlorée en solution dans un solvant organique, par exemple le xylène, à une réaction de greffage à l'aide d'un catalyseur approprié en présence d'au moins un monomère insaturé. Après avoir laissé réagir, à la température appropriée, on introduit éventuellement un catalyseur secondaire et on poursuit la réaction puis on isole le copolymère chloré greffé.

Le catalyseur de greffage peut être choisi par exemple parmi les peroxydes tels que le peroxyde de benzoyle, les perbenzoates tels que le perbenzoate de tertiobutyle, les hydroperoxydes tels que l'hydroperoxyde de dicumyle et les composés diazoïques tels que le 2,2'-azo-bis-(isobutyronitrile), ou le 2,2'-azo-bis-(2-méthylbutyronitrile).

Le catalyseur secondaire éventuellement utilisé dans le procédé est de préférence choisi parmi le peroxyde de dicumyle ou le peroxyde de ditertiobutyle.

La température de la réaction de greffage est généralement supérieure à 120°C mais de préférence inférieure ou égale à 140°C.

Les copolymères chlorés greffés obtenus par le procédé tel que décrit ci-dessus peuvent être isolés ou, de préférence, se présenter sous forme de solutions avec des extraits secs variables pouvant être compris entre 30 et 50%.

On donnera ci-après, à titre d'illustration, un exemple de préparation d'un copolymère chloré greffé.

Les compositions cosmétiques selon l'invention peuvent se présenter sous diverses formes telle que lotions, crèmes, émulsions, gels, solutions, laits, dispersions vésiculaires à base de lipides amphiphiles ioniques ou non-ioniques, lotions de mises en plis ou laques aérosols ou encore sous forme de vernis à ongles.

Dans les compositions selon l'invention, le copolymère chloré greffé peut être présent à une concentration comprise entre 0,01 et 80 % en poids, et de préférence entre 0,5 et 30 % en poids par rapport au poids total de la composition.

Ces compositions peuvent contenir divers adjuvants cosmétiques tels que des corps gras, des solvants organiques, des silicones, des épaississants, des adoucissants, des filtres solaires U.V.-A ou U.V.-B, des agents anti-mousse, des agents hydratants, des humectants, des parfums, des conservateurs, des tensioactifs, des charges, des séquestrants, des émulsionnants, des polymères anioniques, non-ioniques, amphotères ou leurs mélanges, des agents alcalinisants, des colorants, des pigments, des antioxydants, des anti-radicaux libres et tout autre actif habituellement utilisé en cosmétique.

Comme corps gras, on peut en particulier citer les acides gras, les alcools gras, les esters d'acides gras tels que les triglycérides d'acides gras en C₆-C₁₈, la vaseline, la paraffine, la lanoline et la lanoline hydrogénée ou acétylée.

Parmi les huiles, on peut citer les huiles minérales, animales, végétales ou synthétiques et, en particulier, l'huile de vaseline, l'huile de paraffine, l'huile de ricin, l'huile de jojoba, l'huile de sésame ainsi que les huiles et gommes de silicone et les isoparaffines.

Parmi les cires animales, fossiles, végétales, minérales ou de synthèse, on peut mentionner la cire d'abeilles, la cire de caroube, la cire de candelila, l'ozokérite, les cires microcristallines ainsi que les cires et résines de silicone.

Parmi les solvants organiques, ceux préférentiellement utilisés dans les compositions cosmétiques selon l'invention sont les monoalcools ou polyalcools inférieurs en C₁-C₆ tels que l'éthanol, l'isopropanol, le propylèneglycol, le glycérol, le sorbitol, les cétones telles que l'acétone, les esters tels que l'acétate de butyle ou l'acétate d'éthyle et le toluène.

Comme agents épaississants, on peut notamment citer les dérivés de cellulose, les dérivés d'acide polyacrylique réticulé, les gommes de guar ou de caroube ainsi que la gomme de xanthane. Comme agents épaississants, on peut également mentionner les argiles organophiles, par exemple la stéaralkonium hectorite ou la bentone.

Les compositions cosmétiques selon l'invention sous forme de dispersions vésiculaires de lipides amphiphiles ioniques ou non-ioniques peuvent être préparées selon les procédés usuels tels que décrits par exemple dans "Les Liposomes en Biologie Cellulaire et Pharmacologie", Ed. INSERM, John Libbey, Eurotext, (1987), p.6-18.

Selon une forme particulièrement préférée de l'invention, la composition cosmétique se présente sous forme d'un vernis à ongles coloré ou incolore.

Selon cette forme de réalisation, le copolymère chloré greffé est généralement présent en une proportion comprise entre 0,5 et 45 % en poids, et plus particulièrement entre 5 et 25 % en poids par rapport au poids total du vernis.

Comme ceci est bien connu, un vernis à ongles classique se compose essentiellement d'un système solvant, d'une substance filmogène, d'une résine et d'un agent plastifiant.

Selon l'invention, la substance filmogène est constituée par le copolymère chloré greffé utilisé soit seul, soit éventuellement en mélange avec une matière filmogène conventionnelle telle que les nitrocelluloses.

Le système solvant du vernis à ongles est essentiellement constitué par un mélange de divers solvants organiques volatils, ceci en vue d'obtenir des temps de séchage relativement courts. Le système solvant représente généralement de 55 à 85 % en poids du poids total du vernis.

Parmi ces solvants, on peut citer l'acétone, l'acétate d'éthyle, l'acétate de butyle, l'acétate de méthoxy-2-éthyle, la méthyléthylcétone, la méthylisobutylcétone, l'acétate de méthyle, l'acétate d'amyle et l'acétate d'isopropyle.

Le système solvant peut également comprendre un diluant qui est de préférence un hydrocarbure linéaire ou ramifié saturé tel que l'hexane ou l'octane ou encore un hydrocarbure aromatique tel que le toluène ou le xylène dans une proportion généralement comprise entre 10 et 35 % en poids par rapport au poids total du vernis.

Le système solvant peut également contenir d'autres solvants volatils tels que l'éthanol, le n-butanol, le n-propanol ou l'isopropanol ou leurs mélanges ainsi que des alcoxyalcools tels que le méthoxyéthanol, l'éthoxyéthanol et l'éthoxypropanol.

Lorsque le vernis selon l'invention contient une substance filmogène secondaire, il s'agit plus particulièrement des nitrocelluloses du type "RS" ou "SS" en particulier la nitrocellulose type 1/4 seconde RS, la nitrocellulose type 1/2 seconde SS et la nitrocellulose type 5/6 seconde RS.

D'autres substances filmogènes peuvent éventuellement être utilisées, par exemple des dérivés polyvinyliques tels que le butyrate de polyvinyle ainsi que les copolymères décrits dans les brevets français FR-A-2 478 998 , FR-A-2 499 851 et FR-A-2 617 043.

Dans les vernis à ongles selon l'invention, la résine est généralement présente à une concentration comprise entre 0,5 et 15 % en poids par rapport au poids total du vernis.

Parmi les nombreuses résines utilisables, on peut notamment mentionner les résines du type arylsulfonamide formaldéhyde ou arylsulfonamide époxy, notamment la résine toluène sulfonamide formaldéhyde plus connue sous les dénominations commerciales de "Santolite MHP", "Santolite MS 80%" et "Ketjenflex MS 80" ou encore des résines alkydes comme celles vendues par la Société Dainippon sous la dénomination "Beckosol Ode 230-70" et les résines acryliques telles que celles vendues par la Société Rohm & Haas sous la dénomination de "Acryloid B66".

L'agent plastifiant du vernis à ongles selon l'invention est généralement présent à une concentration comprise entre 2 et 10 % en poids par rapport au poids total du vernis.

Les agents plastifiants particulièrement préférés selon l'invention sont choisis de préférence parmi : le phosphate de tricrésyle, le benzoate de benzyle, le phosphate de tributyle, l'acétylricinoléate de butyle, l'acétylricinoléate de glycéryle, le phtalate de dibutyle, le glycolate de butyle, le phtalate de dioctyle, le stéarate de butyle, le phosphate de tributoxyéthyle, le phosphate de triphényle, le citrate de triéthyle, le citrate de tributyle, l'acétylcitrate de tributyle, l'acétylcitrate de triéthyl-2 hexyle, le tartrate de dibutyle, le phtalate de diméthoxyéthyle, le phtalate de diisobutyle, le phtalate de diamyle, le camphre, le triacétate de glycérol et leurs mélanges.

Lorsque le vernis à ongles selon l'invention se présente sous forme colorée, il contient alors au moins un pigment de nature organique ou inorganique.

Parmi les pigments organiques, on peut citer les D & C Red n° 10, 11, 12 et 13, le D & C Red n°7, les D & C Red n°5 et 6, les D & C Red n°34, des laques telles que la laque D & C Yellow n°5 et la laque D & C Red n°2 et la guanine.

Parmi les pigments inorganiques, on peut citer le dioxyde de titane, l'oxychlorure de bismuth, l'oxyde de fer brun et les oxydes de fer rouge ainsi que les agents nacrants synthétiques tels que le mica-titane. Ces pigments peuvent avoir subi un traitement de surface minéral ou organique et en particulier avec une silicone ou un polyéthylène.

Les pigments sont généralement présents dans le vernis à ongles en une proportion comprise entre 0,01 et 2 % en poids par rapport au poids total du vernis.

En vue d'éviter la sédimentation des pigments, certains agents thixotropes peuvent être employés, tels que par exemple la "Bentone 27" ou la "Bentone 38". On peut également ajouter aux vernis des adjuvants de rhéologie tels que de l'acide citrique, de l'acide phosphorique, de l'acide paratoluène sulfonique ou encore de l'acide malique.

Les vernis à ongles selon l'invention peuvent également contenir des adjuvants usuels tels que, par exemple, des filtres U.V., en particulier la benzophénone ou le 2-cyano-3,3-diphénylacrylate d'éthyle, du formol, des vitamines telles que l'acétate de vitamine E et le panthénol, des agents renforçateurs et des antimycotiques.

On va maintenant donner à titre d'illustration des exemples de préparation de copolymères chlorés greffés ainsi que de compositions cosmétiques les contenant.

### EXEMPLE DE PREPARATION DE COPOLYMERES CHLORES GREFFES

### EXEMPLE 1 :

Dans un réacteur de 20 litres muni d'une agitation et d'un dispositif de purge par azote, on introduit une solution de polyéthylène chloré constituée de 1,34 kg d'une solution de polyoléfine chlorée à 50 % dans le xylène vendue sous la dénomination de "CP343" par la Société Kodak (poids moléculaire : 18.000) et 0,48 kg d'acétate de butyle. On porte la température à 135°C sous azote, puis on ajoute séparément un mélange de monomères constitué de 2,8 kg de styrène, de 2,9 kg de méthacrylate de butyle, de 1,16 kg de méthacrylate de 2-hydroxyéthyle, de 0,44 kg d'acide méthacrylique et le catalyseur de polymérisation constitué d'un mélange de 0,04 kg d'azo-bis-isobutyronitrile et de 2,54 kg d'acétate de butyle. L'addition est réalisée en 2h30, temps pendant lequel le contenu du réacteur est maintenu à une température de 134-135°C. On ajoute ensuite, en une heure environ, le catalyseur secondaire constitué de 0,06 kg de peroxyde de tertiobutyle et de 4,7 kg d'acétate de butyle. On laisse la réaction se poursuivre pendant 4 heures à 135°C, puis on refroidit à une température inférieure à 75°C pour introduire le solvant de dilution, à savoir 3,56 kg d'acétate d'éthyle. Après refroidissement, on obtient un produit visqueux parfaitement transparent qui présente les caractéristiques suivantes :

| | |
|---|---|
| - Extrait sec à 90°C | 43,8 % |
| - Transparence | Excellente |
| - Taux de monomères résiduels | 0,21 % |

### EXEMPLES DE COMPOSITION

### EXEMPLE A : Vernis à ongles

| | |
|---|---|
| - Copolymère chloré greffé obtenu selon l'Exemple 1 | 13 % |
| - Nitrocellulose | 4,9 % |
| - Alcool isopropylique | 2,1 % |
| - Acétate d'éthyle | 13 % |
| - Acétate de butyle | 49 % |
| - Formol (Solution à 40%) | 0,4 % |
| - Acétyltributyle citrate ("Citroflex A4" vendu par la Société Pfizer) | 7 % |
| - Camphre | 0,5 % |
| - Pigments | 0,1 % |

Le vernis obtenu est brillant et a une bonne tenue.

### EXEMPLE B : Vernis à ongles

| | |
|---|---|
| - Copolymère chloré greffé obtenu selon l'Exemple 1 | 23 % |
| - Heptane | 10 % |
| - Acétate d'éthyle | 20 % |
| - Acétate de butyle | 34,9 % |
| - Acétyltributyle citrate ("Citroflex A4" vendu par la Société Pfizer) | 10 % |
| - DC Red 6 Ba lake | 0,3 % |
| - DC Red 7 Al lake | 0,2 % |
| - TiO₂ | 0,3 % |
| - Stéaralkonium hectorite | 1,2 % |
| - Acide citrique | 0,1 % |

Le vernis obtenu est brillant et adhère bien sur l'ongle.

### EXEMPLE C : Spray en flacon pompe

| | |
|---|---|
| - Copolymère chloré greffé obtenu selon l'Exemple 1 | 7 % |
| - Amino-2 méthyl-2 propanol-1 | q.s.p. 100 % de neutralisation |
| - Parfum | q.s. |
| - Ethanol | q.s.p. 100 g |

### EXEMPLE D : Spray aérosol

Dans un récipient aérosol, on introduit 40g de la composition de l'exemple C puis un mélange propulseur constitué par :

| | |
|---|---|
| - Diméthyléther | 40 g |
| - Pentane | 20 g |

Après fermeture du récipient, adaptation d'une valve appropriée et mise sous pression, on obtient par pulvérisation un excellent spray donnant un bon pouvoir de fixation aux cheveux.

## Revendications

1. Composition cosmétique filmogène, caractérisée par le fait qu'elle contient dans un véhicule cosmétique approprié au moins un copolymère chloré greffé résultant du greffage de 5 à 20 % d'une polyoléfine chlorée avec 80 à 95 % de monomères insaturés du type acrylique, styrénique et/ou vinylique, monomères pouvant choisis parmi les monomères oléophiles en une proportion maximum de 95 % et les monomères hydrophiles en une proportion maximum de 40 %.

2. Composition cosmétique selon la revendication 1, caractérisée par le fait que la polyoléfine chlorée a un poids moléculaire moyen compris entre 8.000 et 140.000 et une teneur en chlore comprise entre 15 et 40 %.

3. Composition cosmétique selon la revendication 1 ou 2, caractérisée par le fait que les monomères sont du type oléophile et sont choisis parmi le styrène, les acrylates et méthacrylates d'alkyle.

4. Composition cosmétique selon la revendication 1 ou 2, caractérisée par le fait que les monomères insaturés sont du type hydrophile et sont choisis parmi l'acide acrylique, l'acide méthacrylique, les acrylates ou méthacrylates de 2-hydroxyéthyle ou de 2-hydroxypropyle.

5. Composition cosmétique selon l'une quelconque des revendications 1 à 4, caractérisée par le fait que les monomères insaturés se présentent sous forme d'un mélange constitué de styrène, de méthacrylate de butyle, d'acide méthacrylique et de méthacrylate de 2-hydroxyéthyle.

6. Composition cosmétique selon l'une quelconque des revendications précédentes, caractérisée par le fait que le copolymère chloré greffé est présent à une concentration comprise entre 0,01 et 80 %, et de préférence entre 0,5 et 30 % en poids par rapport au poids total de la composition.

7. Composition cosmétique selon l'une quelconque des revendications précédentes, caractérisée par le fait qu'elle se présente sous la forme d'une crème, d'une émulsion, d'un gel, d'une solution, d'un lait, d'une dispersion vésiculaire à base de lipide amphiphile ionique ou non-ionique, d'une lotion de mises en plis ou d'une laque aérosol.

8. Composition selon l'une quelconque des revendications précédentes, caractérisée par le fait qu'elle contient en outre des corps gras, des solvants organiques, des silicones, des épaississants, des filtres solaires U.V.-A ou U.V.-B, des agents anti-mousse, des agents hydratants, des humectants, des parfums, des conservateurs, des tensioactifs, des charges, des séquestrants, des émulsionnants, des polymères anioniques, non-ioniques, amphotères ou leurs mélanges, des agents alcalinisants, des colorants, des pigments, des antioxydants, des anti-radicaux libres, et tout autre actif usuel en cosmétique.

9. Vernis à ongles, coloré ou incolore, constitué d'un système solvant, d'une substance filmogène, d'une résine et d'un agent plastifiant, caractérisé par le fait qu'il contient en tant que substance filmogène principale un copolymère chloré greffé résultant du greffage de 5 à 20 % d'une polyoléfine chlorée, avec 80 à 95 % de monomères insaturés du type acrylique, styrénique et/ou vinylique, monomères pouvant être choisis entre les monomères oléophiles en une proportion maximum de 95 % et lés monomères hydrophiles en une proportion maximum de 40 %.

10. Vernis à ongles selon la revendication 9, caractérisé par le fait que le copolymère chloré greffé est présent en une proportion comprise entre 0,5 et 45 % en poids et plus particulièrement entre 5 et 25 % en poids par rapport au poids total du vernis.

11. Vernis à ongles selon les revendications 9 et 10, caractérisé par le fait qu'il contient, à titre de substance filmogène secondaire, de la nitrocellulose.

12. Vernis à ongles selon l'une quelconque des revendications 9 à 11, caractérisé par le fait qu'il contient une résine choisie parmi les résines du type arylsulfonamide formaldéhyde ou arylsulfonamide époxy, une résine alkyde ou une résine acrylique en une proportion comprise entre 0,5 et 15 % en poids par rapport au poids total du vernis.

13. Vernis à ongles selon l'une quelconque des revendications 9 à 12, caractérisé par le fait que l'agent plastifiant est présent en une concentration comprise entre 2 et 10 % en poids par rapport au poids total du vernis.

14. Vernis à ongles selon l'une quelconque des revendications 9 à 13, caractérisé par le fait qu'il contient au moins un pigment de nature organique ou inorganique en une proportion comprise entre 0,01 et 2 % en poids par rapport au poids total du vernis.

15. Vernis à ongles selon l'une quelconque des revendications 9 à 14, caractérisé par le fait qu'il contient en outre au moins un filtre U.V., du formol, une vitamine telle que l'acétate de vitamine E ou du panthénol, un agent renforçateur ou un agent antimycotique.

## Claims

1. Film-forming cosmetic composition, characterized in that it contains, in a suitable cosmetic vehicle, at least one chlorinated graft copolymer resulting from the grafting of 5 to 20 % of a chlorinated polyolefin with 80 to 95 % of acrylic, styrene and/or vinyl type unsaturated monomers, it being possible for the monomers to be chosen from oleophilic monomers, in a maximum proportion of 95%, and hydrophilic monomers, in a maximum proportion of 40%.

2. Cosmetic composition according to Claim 1, characterized in that the chlorinated polyolefin has an average molecular weight of between 8,000 and 140,000 and a chlorine content of between 15 and 40 %.

3. Cosmetic composition according to Claim 1 or 2, characterized in that the monomers are of the oleophilic type and are chosen from styrene and alkyl acrylates and methacrylates.

4. Cosmetic composition according to Claim 1 or 2, characterized in that the unsaturated monomers are of the hydrophilic type and are chosen from acrylic acid, methacrylic acid and 2-hydroxyethyl or 2-hydroxypropyl acrylates or methacrylates.

5. Cosmetic composition according to any one of Claims 1 to 4, characterized in that the unsaturated monomers take the form of a mixture consisting of styrene, butyl methacrylate, methacrylic acid and 2-hydroxyethyl methacrylate.

6. Cosmetic composition according to any one of the preceding claims, characterized in that the chlorinated graft copolymer is present at a concentration of between 0.01 and 80 %, and preferably between 0.5 and 30 %, by weight relative to the total weight of the composition.

7. Cosmetic composition according to any one of the preceding claims, characterized in that it takes the form of a cream, emulsion, gel, solution, milk, vesicular dispersion based on ionic or nonionic amphiphilic lipid, a setting lotion or an aerosol lacquer.

8. Composition according to any one of the preceding claims, characterized in that it contains, in addition, fats, organic solvents, silicones, thickeners, UV-A or UV-B sunscreen agents, antifoams, hydrating agents, humectants, perfumes, preservatives, surfactants, fillers, sequestering agents, emulsifiers, anionic, nonionic and amphoteric polymers or mixtures thereof, alkalinizing agents, colorants, pigments, antioxidants, anti-free-radical agents and any other active agent commonly used in cosmetics.

9. Coloured or colourless nail varnish consisting of a solvent system, a film-forming substance, a resin and a plasticizing agent, characterized in that it contains as main film-forming substance a chlorinated graft copolymer resulting from the grafting of 5 to 20 % of a chlorinated polyolefin with 80 to 95 % of acrylic, styrene and/or vinyl type unsaturated monomers, it being possible for the monomers to be chosen from oleophilic monomers, taken separately or mixed in a proportion of 0 to 95 %, and hydrophilic monomers, also taken separately or mixed in a proportion of 0 to 40 %.

10. Nail varnish according to Claim 9, characterized in that the chlorinated graft copolymer is present in a proportion of between 0.5 and 45 % by weight, and more especially between 5 and 25 % by weight, relative to the total weight of the varnish.

11. Nail varnish according to Claims 9 and 10, characterized in that it contains nitrocellulose as secondary film-forming substance.

12. Nail varnish according to any one of Claims 9 to 11, characterized in that it contains a resin chosen from arylsulphonamide/formaldehyde or arylsulphonamide/epoxy type resins, an alkyde resin or an acrylic resin, in a proportion of between 0.5 and 15 % by weight relative to the total weight of the varnish.

13. Nail varnish according to any one of Claims 9 to 12, characterized in that the plasticizing agent is present in a concentration of between 2 and 10 % by weight relative to the total weight of the varnish.

14. Nail varnish according to any one of Claims 9 to 13, characterized in that it contains at least one pigment of organic or inorganic nature in a proportion of between 0.01 and 2 % by weight relative to the total weight of the varnish.

15. Nail varnish according to any one of Claims 9 to 14, characterized in that it contains, in addition, at least one UV screening agent, formaldehyde, a vitamin such as vitamin E acetate or pantothenol, a strengthening agent or an antimycotic agent.

## Patentansprüche

1. Filmbildende kosmetische Zubereitung, dadurch gekennzeichnet, daß sie in einem geeigneten kosmetischen Träger mindestens ein gepfropftes, chloriertes Copolymer enthält, das aus der Pfropfung von 5 bis 20 % eines chlorierten Polyolefins mit 80 bis 95 % ungesättigten Monomeren von Acrylsäure-, Styrol- und/oder Vinyltyp stammt, wobei die Monomeren unter oleophilen Monomeren in einem höchsten Mengenverhältnis bis 95% und unter hydrophilen Monomeren, in einem höchsten Mengenverhältnis bis 40% ausgewählt sein können.

2. Kosmetische Zubereitung nach Anspruch 1, dadurch gekennzeichnet, daß das chlorierte Polyolefin ein durchschnittliches Molekulargewicht aufweist, das zwischen 8 000 und 140 000 beträgt, wobei ein Chlorgehalt zwischen 15 und 40 % vorhanden ist.

3. Kosmetische Zubereitung nach Anspruch 1 oder 2, dadurch gekennzeichnet, daß die Monomeren vom oleophilen Typ sind und unter Styrol, Alkylacrylaten und Alkylmethacrylaten ausgewählt sind.

4. Kosmetische Zubereitung nach Anspruch 1 oder 2, dadurch gekennzeichnet, daß die ungesättigten Monomeren vom hydrophilen Typ und unter Acrylsäure, Methacrylsäure, 2-Hydroxyethyl- oder 2-Hydroxypropylacrylaten oder 2-Hydroxyethyl- oder 2-Hydroxypropylmethacrylaten ausgewählt sind.

5. Kosmetische Zubereitung nach einem der Ansprüche 1 bis 4, dadurch gekennzeichnet, daß die ungesättigten Monomeren in Form eines aus Styrol, Butylmethacrylat, Methacrylsäure oder 2-Hydroxyethylmethacrylat bestehenden Gemisches ausgewählt sind.

6. Kosmetische Zubereitung nach einem der vorstehenden Ansprüche, dadurch gekennzeichnet, daß das gepfropfte, chlorierte Copolymer in einer Konzentration zwischen 0,01 und 80 Gew.-%, vorzugsweise zwischen 0,5 und 30 Gew.-% in bezug auf das Gesamtgewicht der Zusammensetzung vorhanden ist.

7. Kosmetische Zubereitung nach einem der vorstehenden Ansprüche, dadurch gekennzeichnet, daß sie in Form einer Creme, Emulsion, eines Gels, einer Lösung, einer Milch, einer vesikulären Dispersion auf Basis eines ionischen oder nichtionischen amphiphilen Lipids, einer Lotion oder eines Lackaerosols vorliegt.

8. Zubereitung nach einem der vorstehenden Ansprüche, dadurch gekennzeichnet, daß sie zusätzlich noch Fettkörper, organische Lösungsmittel, Silikone, Verdickungsmittel, UV-A- oder UV-B-Sonnenfiltersubstanzen, Antischaummittel, Hydratisierungsmittel, Feuchthaltemittel, Duftstoffe, Konservierungsmittel, Tenside, Trägerstoffe, Komplexiermittel, Emulgatoren, anionische, nichtionische, amphotäre Polymere oder deren Gemische, Alkalisierungsmittel, Farbstoffe, Pigmente, Antioxidantien, freie Radikalfänger und jeden anderen in der Kosmetik üblichen Wirkstoff enthält.

9. Farbiger oder farbloser Nagellack, der aus einem Lösungsmittelsystem, einer filmbildenden Substanz, einem Harz und einem Weichmacher besteht, dadurch gekennzeichnet, daß er als hauptsächliche filmbildende Substanz ein gepfropftes chloriertes Copolymer enthält, das aus der Pfropfung von 5 bis 20 % eines chlorierten Polyolefins mit 80 bis 95 % ungesättigten Monomeren vom Acrylsäure-, Styrol- und/oder Vinyltyp stammt, wobei die Monomeren unter oleophilen Monomeren für sich alleine genommen oder in Mischung miteinander in einem Mengenverhältnis von 0 bis 95 % und unter hydrophilen Monomeren für sich alleine genommen oder in Mischung miteinander in einem Mengenverhältnis von 0 bis 40 % ausgewählt sind.

10. Nagellack nach Anspruch 9, dadurch gekennzeichnet, daß das gepfropfte, chlorierte Copolymer in einem Mengenverhältnis zwischen 0,5 und 45 Gew.-%, insbesondere zwischen 5 und 25 Gew.-% in bezug auf das Gesamtgewicht des Lacks vorhanden ist.

11. Nagellack nach den Ansprüchen 9 und 10, dadurch gekennzeichnet, daß er als zweite filmbildende Substanz Nitrocellulose enthält.

12. Nagellack nach einem der Ansprüche 9 bis 11, dadurch gekennzeichnet, daß er ein Harz enthält, das unter Harzen vom Typ Arylsulfonamid/Formaldehyd oder Arylsulfonamid/Epoxy, einem Alkydharz, oder einem Acrylsäureharz in einem Mengenverhältnis zwischen 0,5 und 15 Gew.-% in bezug auf das Gesamtgewicht des Lacks ausgewählt ist.

13. Nagellack nach einem der Ansprüche 9 bis 12, dadurch gekennzeichnet, daß der Weichmacher in einer Konzentration zwischen 2 und 10 Gew.-% in bezug auf das Gesamtgewicht des Lacks vorhanden ist.

14. Nagellack nach einem der Ansprüche 9 bis 13, dadurch gekennzeichnet, daß er mindestens ein Pigment organischen oder anorganischen Ursprungs in einer Menge zwischen 0,01 und 2 Gew.-% in bezug auf das Gesamtgewicht des Lacks enthält.

15. Nagellack nach einem der Ansprüche 9 bis 14, dadurch gekennzeichnet, daß er weiter einen UV-Filter, Formol, ein Vitamin wie beispielsweise Vitamin E oder Panthenol, ein Festigungsmittel oder ein Antimykotikum enthält.
